# EUROPÄISCHE PATENTSCHRIFT

(11) **EP 1 031 332 B1**
(45) Veröffentlichungstag und Bekanntmachung des Hinweises auf die Patenterteilung: **01.10.2003**
(21) Anmeldenummer: 99103377.0
(22) Anmeldetag: 22.02.1999
(51) Int. Cl.: A61F 2/42

(54) **Handgelenkprothese**
Prosthetic wrist joint
Prothèse du poignet

(43) Veröffentlichungstag der Anmeldung: 30.08.2000
(73) Patentinhaber: Waldemar Link (GmbH & Co.), 22339 Hamburg (DE); Thabe, Heiner Dr. med., 55543 Bad Kreuznach (DE)
(72) Erfinder: Keller, Arnold, 23863 Kayhude (DE); Thabe, Heiner Dr. med., 55543 Bad Kreuznach (DE)
(74) Vertreter: Glawe, Delfs, Moll & Partner

(56) Entgegenhaltungen:
- EP-A- 0 532 440
- EP-A- 0 607 748
- WO-A-95/22945
- FR-A- 2 314 702
- US-A- 4 307 473

## Beschreibung

Das menschliche Handgelenk kann Schwenkbewegungen in allen Raumrichtungen durchführen, wobei die Schwenkung in der Dorsal-Palmar-Ebene die wichtigste ist. Bei einer bekannten Handgelenkprothese (DE-U 295 00 476) sind deshalb zwei Gelenke hinter- oder ineinander vorgesehen, von denen das eine als Querachsgelenk ausgebildet ist, das ausschließlich zu einer Scharnierbewegung im Sinne der Dorsal-Palmar-Schwenkung befähigt ist, während das andere als hauptsächlich quer dazu bewegliches, längliches Pfannengelenk ausgebildet ist. Bei instabilem Bandapparat oder gewissen Muskeldysbalancen bevorzugt man Prothesen, bei denen die Querschwenkbarkeit unterdrückt ist, indem lediglich das Querachsgelenk vorgesehen ist (DE-U 70 12 087). Bislang ist es notwendig, sich im einen und anderen Falle für unterschiedliche Fabrikate zu entscheiden, was dann mißlich ist, wenn die Entscheidung erst intraoperativ fallen kann.

Der Erfindung liegt daher die Aufgabe zugrunde, eine Prothese zu schaffen, die eine intraoperative Entscheidung ermöglicht. Die Lösung liegt in den Merkmalen des Anspruchs 1 und vorzugsweise denen der Unteransprüche.

Demgemäß ist der proximale Teil des Querachsgelenks mit dem in der Elle zu verankernden Radialelement lösbar verbunden und selbst alternativ in sich starr ausgebildet oder mit dem weiteren, die Querbewegung erlaubenden Gelenk ausgerüstet. Es ergibt sich damit ein Prothesensystem, das es gestattet, intraoperativ zu entscheiden, ob eine Querbeweglichkeit zugelassen werden soll oder nicht und demzufolge die eine oder die andere der alternativen Ausführungen des proximalen Teils des Querachsgelenks eingesetzt wird, während die übrigen Prothesenteile gleich bleiben. Insbesondere gestattet dieses System es, die jeweils im Knochen zu verankernden Elemente der Prothese zu implantieren, bevor über die Querbeweglichkeit der Prothese entschieden werden muß. Auch ein späterer Austausch wird dadurch ermöglicht.

Die zwischen dem proximalen Teil des Querachsgelenks und dem Radialelement vorgesehenen Verbindungseinrichtungen sind zweckmäßigerweise zur formschlüssigen Kraftübertragung ausgerüstet. Diese sind nach einem weiteren Merkmal der Erfindung im wesentlichen in ulnar-radialer Richtung ineinanderschiebbar. Dadurch wird zum einen eine verläßliche Verbindung erreicht, die unabhängig ist von irgendwelchen Schraub- oder Klemmechanismen. Zum anderen kann der genannte Prothesenteil ohne wesentliche Distraktion zwischen die bereits implantierten carpalen und radialen Elemente eingefügt werden.

Das Querachsgelenk umfaßt auf der einen Seite einen T-Zapfen und auf der anderen Seite einen Lagerteil, der zwei fluchtende Bohrungen zur Aufnahme des T-Zapfens oder seiner Lagerung enthält. Der T-Zapfen wird vom einen Ende her in diese Bohrungen eingeschoben, wobei die äußere dieser beiden Bohrungen in Längsrichtung geschlitzt ist, um den Mittelbolzen des Zapfens durchzulassen. Die Montageöffnung am Ende der Bohrung wird anschließend geschlossen. Erfindungsgemäß kann dafür ein Gewindeverschluß vorgesehen sein.

Die Grenze zwischen den voneinander lösbaren distalen und proximalen Teilen des Querachsgelenks kann mit diesen Lagerflächen zusammenfallen. Da aber die Lagerfunktionen einerseits und die mit der Lösbarkeit bzw. gegenseitigen Fixierbarkeit der Gelenkteile zusammenhängenden Funktionen andererseits nicht unbedingt miteinander harmonieren, kann es zweckmäßiger sein, daß alle lagerbildenden Elemente des Querachsgelenks am distalen Teil angeordnet sind.

Das Carpuselement weist mehrere distal vorstehende, in die Mittelhandknochen einzufügende Zapfen auf, die durch ein Querglied verbunden sind, das im wesentlichen lotrecht zu den Zapfen verläuft. Es ist bekannt, die Gelenkachse des Querachsgelenks in einem spitzen Winkel dazu anzuordnen. Erfindungsgemäß wird dieser Winkel in einem Bereich zwischen 0° und 15°, vorzugsweise in einem engeren Bereich von 5° bis 10° gewählt.

Die Gelenkpfanne des zusätzlichen Universalgelenks ist bei der bekannten Prothese gegenüber der Querrichtung kräftig geneigt angeordnet. Diese Neigung wird erfindungsgemäß zurückgenommen zugunsten einer nahezu symmetrischen Ausführung, in welcher die Randtangente, d. h. die Verbindungslinie der Randkanten der Gelenkfläche der Gelenkpfanne in der Palmar-Ebene, einen Winkel von mindestens 5° und höchstens 25°, vorzugsweise von 10° bis 15°, mit der Radiusquerrichtung bzw. den Komplementärwinkel mit der Längsrichtung eines in der Elle zu fixierenden Schafts einschließt. In der Sagital-Ebene kann dieser Winkel größer sein. Aus der Differenz dieses Winkels und des im vorstehenden Absatz genannten Winkels ergibt sich die Richtungsdifferenz des Carpuselements gegenüber dem Radiuselement, die vorzugsweise zwischen 0° und 10° liegt. Entsprechend liegt der Winkel, den bei der in sich starren Alternative des proximalen Teils des Querachslagers die Lagerachse mit der Radiusquerrichtung einschließt, vorteilhafterweise in derselben Größenordnung.

Die Erfindung wird im folgenden näher unter Bezugnahme auf die Zeichnung erläutert, die ein vorteilhaftes Ausführungsbeispiel der Erfindung veranschaulicht. Es zeigen:
- Fig. 1 u. 2: die Dorsalansicht einer erfindungsgemäßen Prothese der rechten Hand in beiden Ausführungsalternativen in etwa zweifach vergrößertem Maßstab,
- Fig. 3 u. 4: die entsprechenden Seitenansichten in der in Fig. 1 bzw. 2 angegebenen Pfeilrichtung III bzw. IV,
- Fig. 5: eine der Fig. 1 entsprechende Ansicht des Carpalelements mit freigelegtem distalen Teil des Querachsgelenks,
- Fig. 6 u. 7: eine Seitenansicht und Draufsicht der Olive des weiteren Gelenks,
- Fig. 8: eine Schnittansicht des Querachsgelenks in größerem Maßstab,
- Fig. 9 - 11: entsprechende Darstellungen eines ähnlichen Ausführungsbeispiels.

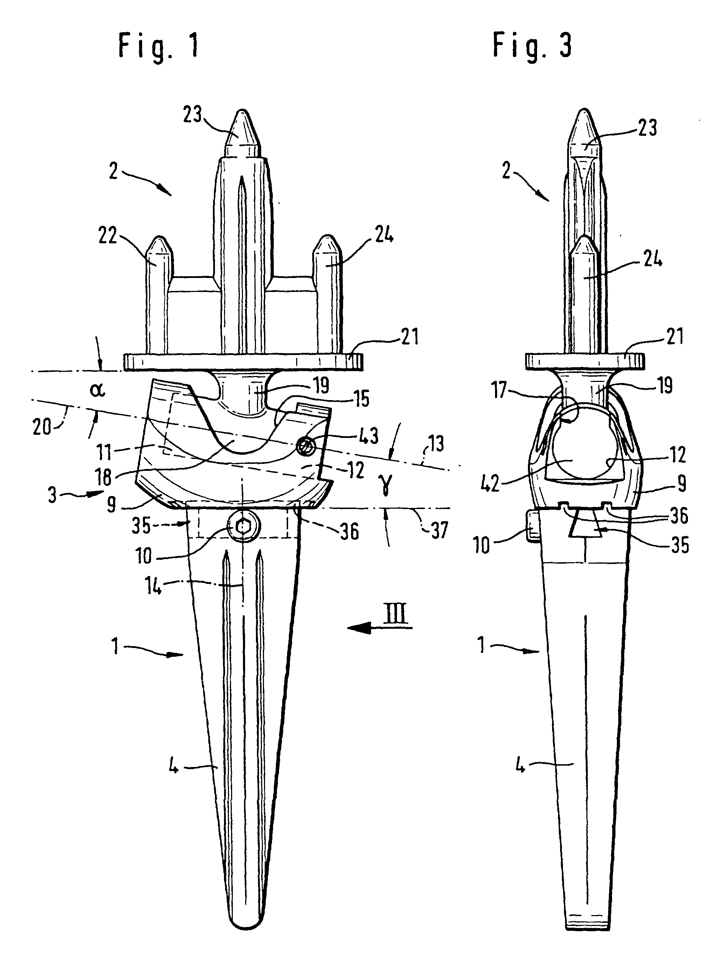

Die Prothese gemäß Fig. 1 umfaßt ein Radialelement 1, ein Carpuselement 2 und ein Querachsgelenk 3. Das Radialelement umfaßt einen Schaft 4, der am distalen Ende der Elle in deren Knochenhöhlung zu implantieren ist. Er endet in einer Verbindungseinrichtung 35 mit etwa in ulnar-radialer Richtung verlaufenden Rippen und Nuten, die schwalbenschwanzförmig ausgebildet sind. Durch Rippeneingriff 36 in entsprechenden Nuten des Gegenstücks werden die Flanken der Schwalbenschwanznut im Falle hoher zu übertragender Kräfte gehindert, aus der Eingriffslage auszuweichen. Nach der Implantation des Schafts 4 ist der proximale Teil 9 des Querachsgelenks 3 mit seiner Verbindungseinrichtung auf diejenige des Schafts von ulnar her aufschiebbar. Die formschlüssig zusammenwirkenden Teile der Verbindungseinrichtungen vermitteln im wesentlichen die gesamte Kraftübertragung. Zur Sicherung des Eingriffs und der korrekten Relativlage kann eine Sicherungseinrichtung, beispielsweise eine Schraube 10, vorgesehen sein.

Der proximale Teil 9 des Querachsgelenks 3 enthält zwei miteinander fluchtende Bohrungsabschnitte 11,12, deren Achse 13 etwa in ulnar-radialer Richtung, d.h. etwa lotrecht zur Mittelachse 14 des Schafts 4 in der Palmar-Ebene, verläuft. Zwischen den Bohrungsabschnitten 11 und 12 befindet sich ein Ausschnitt 15, der bis 70° Flexion und Extension erlaubt. Das Ende des Bohrungsabschnitts 11 ist geschlossen. Das äußere Ende des Bohrungsabschnitts 12 ist zunächst offen. An irgendeiner Seite, vorzugsweise distal, ist der Bohrungsabschnitt 12 bei 17 geschlitzt.

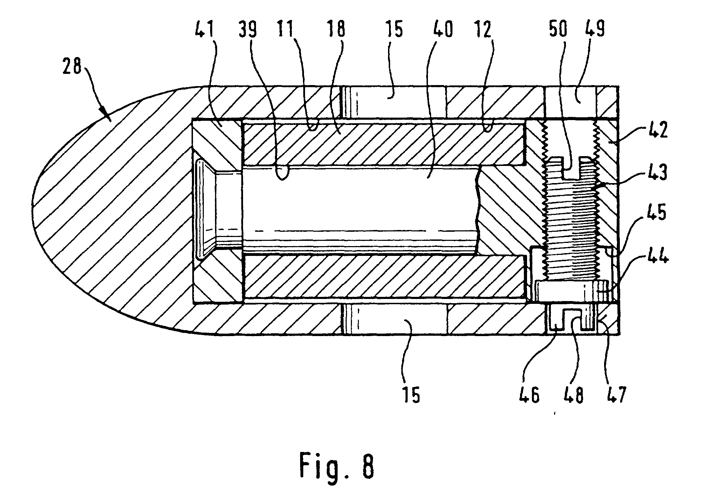

Der distale Teil 18 des Querachsgelenks 3 wird von einem T-Zapfen 18,19 gebildet, dessen Länge und Durchmesser zu den Bohrungsabschnitten 11,12 paßt und dessen Halsstück 19 im montierten Zustand innerhalb des Ausschnitts 15 verschwenkbar ist. Einzelheiten der Ausführungen werden später unter Bezugnahme auf Fig. 8 erläutert. Bei der Montage wird der Zapfen 18 von der offenen Seite her in die Bohrung 11,12 eingeschoben, wobei das Halsstück 19 durch den Schlitz 17 des Bohrungsabschnitts 12 gleitet. Da die Achse 13 und die Richtung der Rippen und Nuten 5 bis 8 etwa parallel verlaufen, kann nach der Implantation des Carpuselements 2 und des Radialelements 1 beim Einschieben des proximalen Teils 9 des Querachsgelenks 3 dieser gleichzeitig mit der Verbindungseinrichtung 35 des Radialelements 1 und dem Zapfen 18 in Eingriff gelangen.

Das Halsstück 19 des T-Zapfens ist starr verbunden mit einem Querstück 21 des Carpuselements 2, von dem die Zapfen 22,23,24 des Carpuselements 2 in distaler Richtung ausgehen. Sie liegen in einer Palmar-Ebene und sind in den Mittelhandknochen zu verankern. Das Querstück 21 des Carpuselements 2 kann plattenförmig ausgebildet sein. Quer zur Palmar-Ebene sollen seine Dimensionen so reduziert sein, daß es den Verlauf der Sehnen und Nerven nicht behindert.

Die Achse 20 des Zapfens 18 verläuft in einem spitzen Winkel a zum Querstück 21 des Carpuselements 2. Dieser Winkel beträgt im dargestellten Beispiel etwa 10°. Das Querstück 21 verläuft unter 90° zu den Zapfen 22 bis 24. Die Bohrungen 11, 12 im proximalen Teil des Querachsgelenks 3 verlaufen zur Querrichtung 37 des Radialelements 1 ebenfalls unter einem Winkel γ von etwa 10°, so daß die Längsrichtungen der Zapfen 22 bis 24 einerseits und des Zapfens 4 andererseits miteinander fluchten bzw. parallel zueinander sind.

Es ist ohne weiteres erkennbar, daß die Prothese gemäß Fig. 1 eine Schwenkbewegung lediglich um die Achse 13 des Querachsgelenks gestattet. Diese Prothese eignet sich daher insbesondere in solchen Fällen, in denen die natürliche Beherrschung der übrigen Freiheitsgrade der natürlichen Hand durch den Bandapparat bzw. die zugehörigen Muskeln nicht mehr möglich ist.

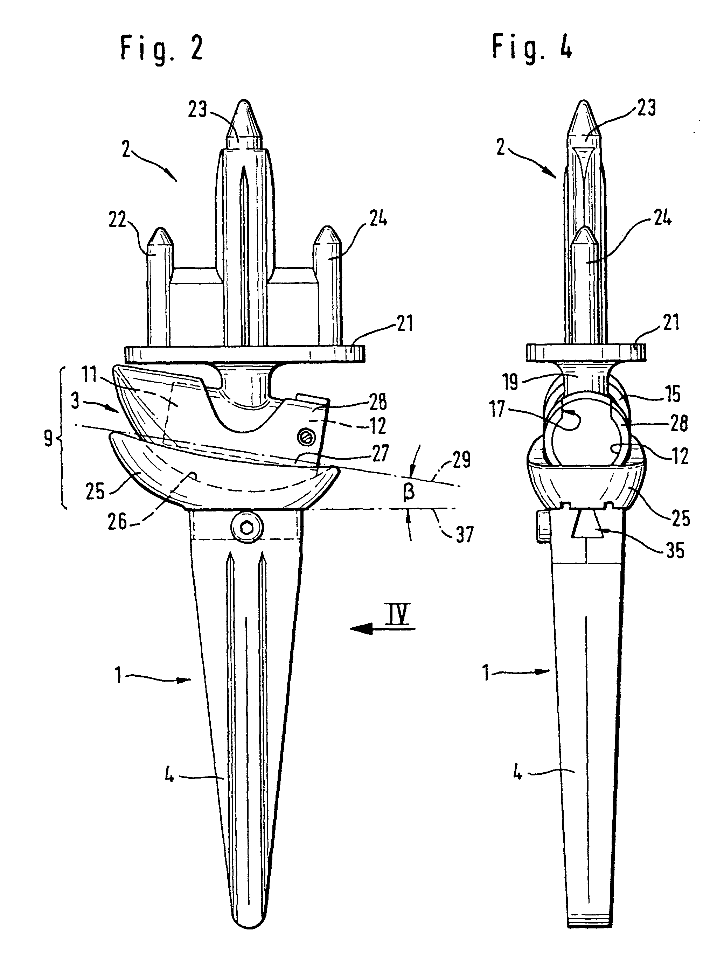

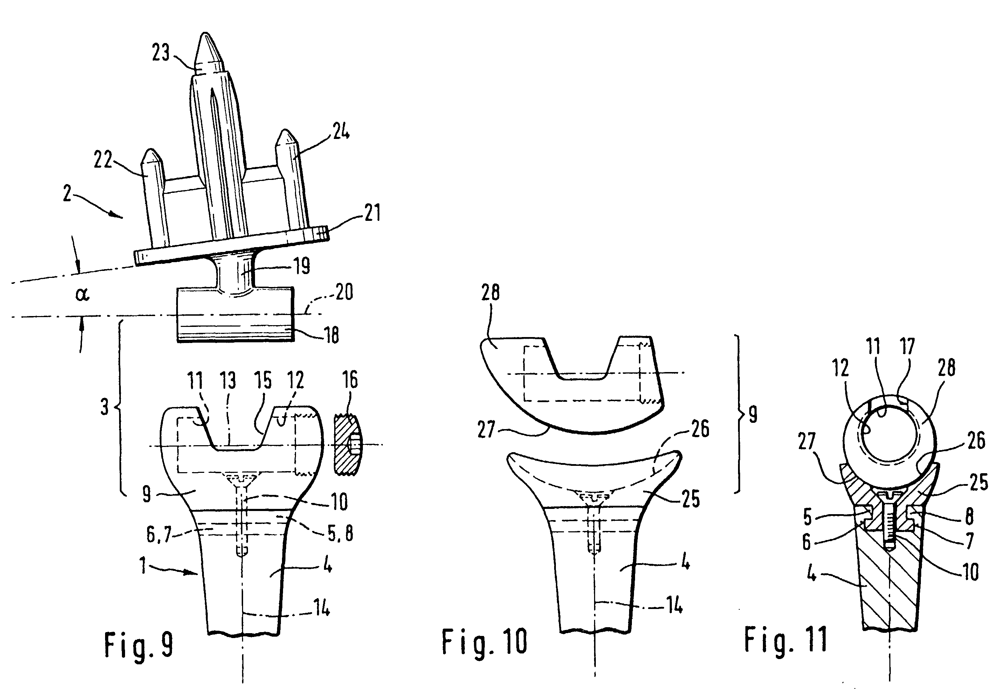

Die lösbare, starre Verbindung zwischen dem Schaft 4 und dem proximalen Teil 9 des Querachsgelenks 3 erlaubt es, diesen Teil 9 gegen den in Fig. 2 und 4 an entsprechender Stelle dargestellten Teil auszutauschen. Die Ausführung gemäß Fig. 2 stimmt mit derjenigen gemäß Fig. 1 völlig überein bis auf das Vorhandensein eines geänderten proximalen Teils 9 des Querachsgelenks. Dieser wird gebildet von einer Pfanne 25, die eine nach distal blickende, konkave Gelenkfläche 26 bildet, und einer dazu komplementären, nach proximal blickenden, konvexen Gelenkfläche 27 an der Olive 28, in der die Bohrungsabschnitte 11,12 für das Querachslager enthalten sind. Der Pfannenteil kann in seiner Höhe variabel gestaltet werden, um z.B. carpale Höhenminderungen durch Knochendefekte zusätzlich auszugleichen. Die Gelenkflächen 26,27 sind vorzugsweise nicht sphärisch, sondern in ulnar-radialer Richtung länglich. In der Palmar-Ebene (Fig. 2) sind der Krümmungsradius und die Ausdehnung der Gelenkflächen größer als in der Sagittal-Ebene (Fig. 11). Der Winkel β, den die Randtangente 29 mit der Querrichtung des Radialelements bildet, liegt um 10° bis 15°.

Die Gelenkflächen 26,27 erlauben eine Schwenkbewegung (Abund Adduktion) in der Palmar-Ebene. Damit dies möglich ist, stimmen ihre Querschnittsformen, gesehen in auf die gemeinsame Schwenkachse bezogenen Radialebenen, im wesentlichen überein. Da die Krümmungsradien quer zur Palmar-Ebene wesentlich kleiner sind als der Krümmungsradius in der Palmar-Ebene, können sie bei einer palmar-dorsalen Schwenkbewegung (für die das Querachsgelenk bestimmt ist) nicht unter Beibehaltung ihrer vollflächigen Anlage aufeinander gleiten und widerstreben daher einer solchen Bewegung. Aber sie verhalten sich bei der Beanspruchung nicht starr und erlauben daher einen gewissen Grad von Pronation bzw. Supination. Auch gegenüber Drehbeanspruchung sind sie nicht völlig starr. Dies kommt dem Bestreben entgegen, die Prothesenkomponenten, falls möglich, nicht völlig starr miteinander zu verbinden, um ihre Verankerung im Knochen von Kraftspitzen zu entlasten.

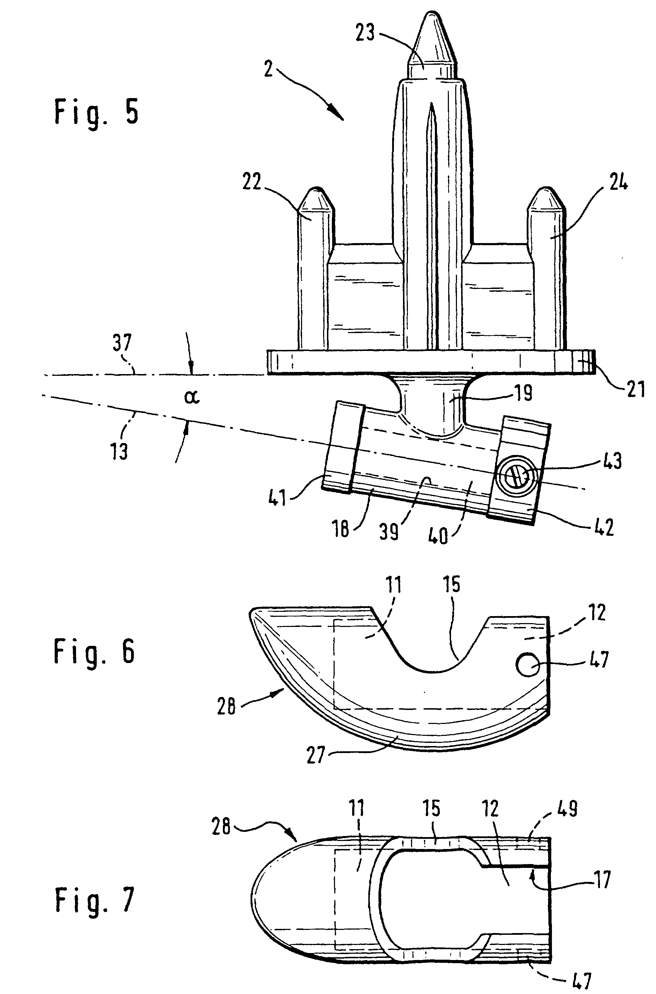

Wie man Fig. 5 und 8 entnimmt, enthält der Zapfen 18 des distalen Querachsgelenks eine Lagerbohrung 39, die eine Achse 40 aufnimmt, die starr mit zwei Endstücken 41,42 verbunden ist, die sich beiderseits des Zapfens 18 befinden und die Achse 40 darin sichern. Die Außendurchmesser der Endstücke 41,42 sind ein wenig größer als der Außendurchmesser des Zapfens 18 und gleichen mit geringem Untermaß dem Durchmesser der Bohrung 11,12 im proximalen Teil 9 des Querachslagers. In dieser Ausführung der Prothese liegt also nicht der Teil 18 an der Innenfläche der Bohrung 39 an, sondern die Endstücke 41,42. Dies gestattet es, die Bohrung 11,12 von der Lagerfunktion zu befreien. Diese wird vielmehr von der Achse 40 in der Bohrung 39 übernommen, wobei die Endstücke 41,42 die Axialkräfte übernehmen.

Zur Sicherung des Zapfens 18 und der zugehörigen Teile in der Bohrung 11,12 und zur Übertragung der Axialkräfte auf den proximalen Teil des Querachsgelenks ist eine Sicherungsschraube 43 in dem Endstück 42 quer angeordnet (Fig. 8). Ihre Gesamtlänge ist geringer als der Durchmesser dieses Endstücks 42, so daß sie die Montage nicht behindert, wenn sie mit ihrem Kragen 44 bis gegen einen entsprechenden Anschlag 45 in der im Endstück 42 enthaltenen Bohrung geschraubt ist. Nach der Montage wird sie so weit herausgedreht, daß ihr Kopfansatz 46 in die damit fluchtende Bohrung 47 der Olive 28 bzw. des proximalen Teils 9 des Querachsgelenks eindringt. Zu diesem Zweck ist der Kopf 46 mit Schlüsselflächen 48 versehen, die durch die Bohrung 47 hindurch zugänglich sind, wenn die Prothese für die rechte Hand benutzt wird. Im Falle der Operation der linken Hand ist die gegenüberliegende Seite der Prothese dem Operateur zugewendet. Deshalb ist eine entsprechende Bohrung 49 auf der gegenüberliegenden Seite vorgesehen, durch die am Schraubenfuß vorgesehene Schlüsselflächen 50 zugänglich werden.

Das weitere Ausführungsbeispiel gemäß Fig. 9 bis 11 gleicht, soweit im folgenden nicht ausdrücklich erwähnt, dem zuvor beschriebenen.

Das offene Ende der Bohrung 11,12 ist mit einem Gewinde versehen, das den Verschluß mittels eines Gewindestopfens 16 gestattet. Statt dessen kann auch ein Sprengring oder dergleichen vorgesehen werden.

Die Achse 13 des Querachsgelenks ist in der Ausführung gemäß Fig. 9 etwa lotrecht zur Achse 14 des Radialelements angeordnet. Daraus folgt, daß das Carpuselement mit seinen Zapfen 22 bis 24 einerseits und das Radialelement 4 andererseits schwach winklig zueinander eingestellt sind.

Entsprechend ist die Pfanne 25,26 in der Ausführung gemäß Fig. 10 symmetrisch zur Achse 14 des Radialelements ausgebildet.

Für die Verbindungseinrichtungen zwischen dem proximalen Teil 9 des Querachsgelenks und dem Radialelement 14 ist eine von der zuvor erläuterten Ausführungsform abweichende Anordnung von Rippen 5,8 und Nuten 6,7 am Radialelement vorgesehen, die mit entsprechenden Rippen und Nuten des proximalen Querachsteils zusammenwirken.

Schließlich besteht ein Unterschied dieses Ausführungsbeispiels gegenüber dem zuvor erläuterten darin, daß der Zapfen 18 des Querachsgelenks einerseits und die Bohrung 11,12 andererseits unmittelbar die aufeinander gleitenden Gelenkflächen bilden.

## Patentansprüche

1. Handgelenkprothese mit einem in den Mittelhandknochen zu implantierenden Carpuselement (2), einem am Unterarmknochen zu fixierenden Radialelement (1) und einem dazwischen angeordneten Querachsgelenk (3), dessen proximaler Teil (9) mit dem Radialelement (1) verbunden ist, **dadurch gekennzeichnet, daß** zwei Ausführungen des proximalen Teils (9) des Querachsgelenks (3) verfügbar sind, von denen die erste in sich starr ist und die zweite ein weiteres Gelenk (25,28) enthält, und daß beide Ausführungen des proximalen Teils (9) des Querachsgelenks (3) lösbar mit dem Radialelement (4) und dem distalen Teil (18) des Querachsgelenks (3) verbunden bzw. verbindbar sind.

2. Prothese nach Anspruch 1, **dadurch gekennzeichnet, daß** der proximale Teil (9) des Querachsgelenks (3) und das Radialelement (1) mit formschlüssig kraftübertragenden Verbindungseinrichtungen (5-8) versehen sind.

3. Prothese nach Anspruch 1 oder 2, **dadurch gekennzeichnet, daß** das Querachsgelenk (3) einen T-Zapfen (18,19) umfaßt, der den distalen Teil (18) des Querachsgelenks (3) bildet.

4. Prothese nach einem der Ansprüche 1 bis 3, **dadurch gekennzeichnet, daß** der distale Teil (18) des Querachsgelenks (3) die lagerbildenden Elemente (41,42) des Querachsgelenks (3) umfaßt und die Verbindung des distalen Teils (18) des Querachsgelenks (3) mit dem proximalen Teil (9) des Querachsgelenks (3) statisch ist.

5. Prothese nach einem der Ansprüche 1 bis 4, **dadurch gekennzeichnet, daß** der Winkel (α) zwischen der Achse (13) des Querachsgelenks (3) und einem Querstück (21) des Carpuselements (2) nicht größer als 15° ist.

6. Prothese nach Anspruch 5, **dadurch gekennzeichnet, daß** das zusätzliche Gelenk (25,28) eine Gelenkpfanne (25) umfaßt, deren Randtangente (29) in der Palmar-Ebene einen Winkel (β) von 5° bis 25° mit der Radiusquerrichtung (37) einschließt.

7. Prothese nach einem der Ansprüche 1 bis 6, **dadurch gekennzeichnet, daß** der proximale Teil (9) des Querachsgelenks (3) eine ulnar-radial verlaufende Bohrung (11,12) zur Aufnahme des distalen Teils (18) des Querachsgelenks (3) bzw. seiner lagerbildenden Elemente (40,41,42) enthält, deren Öffnung ein Verschlußelement (42) aufnimmt, das eine mit einer Ausnehmung (47) in der Bohrungswand zusammenwirkende Sicherungsschraube (43) enthält, die an ihren beiden Enden durch Öffnungen (47,49) zugängliche Schlüsselflächen (48,50) aufweist.

8. Prothese nach Anspruch 7, **dadurch gekennzeichnet, daß** das Verschlußelement (42) von dem Endstück eines der lagerbildenden Elemente (41,42) gebildet ist.

9. Prothese nach einem der Ansprüche 2 bis 8, **dadurch gekennzeichnet, daß** die Verbindungseinrichtungen (5-8) und der proximale (9) und distale (18) Teil des Querachsgelenks (3) in derselben Richtung ineinanderschiebbar sind.

## Claims

1. Wrist prosthesis with a carpus element (2) to be implanted in the metacarpal bones, a radial element (1) to be fixed on the bone of the forearm, and, arranged between these, a transverse axis articulation (3) whose proximal part (9) is connected to the radial element (1), **characterized in that** two embodiments of the proximal part (9) of the transverse axis articulation (3) are available, the first of which is rigid per se and the second of which comprises a further articulation (25, 28), and **in that** both embodiments of the proximal part (9) of the transverse axis articulation (3) are connected or can be connected releasably to the radial element (4) [sic] and to the distal part (18) of the transverse axis articulation (3).

2. Prosthesis according to Claim 1, **characterized in that** the proximal part (9) of the transverse axis articulation (3) and the radial element (1) are provided with connecting arrangements (5 - 8) which transmit force with positive locking.

3. Prosthesis according to Claim 1 or 2, **characterized in that** the transverse axis articulation (3) comprises a T-type journal (18, 19) which forms the distal part (18) of the transverse axis articulation (3).

4. Prosthesis according to one of Claims 1 to 3, **characterized in that** the distal part (18) of the transverse axis articulation (3) comprises the bearing elements (41, 42) of the transverse axis articulation (3), and the connection of the distal part (18) of the transverse axis articulation (3) to the proximal part (9) of the transverse axis articulation (3) is static.

5. Prosthesis according to one of Claims 1 to 4, **characterized in that** the angle (α) between the axis (13) of the transverse axis articulation (3) and a crosspiece (21) of the carpus element (2) is no greater than 15°.

6. Prosthesis according to Claim 5, **characterized in that** the additional articulation (25, 28) comprises a socket (25) whose edge tangent (29) encloses in the palmar plane an angle (β) of 5° to 25° with the radius transverse direction (37).

7. Prosthesis according to one of Claims 1 to 6, **characterized in that** the proximal part (9) of the transverse axis articulation (3) has a bore (11, 12) extending in the ulnoradial direction and receiving the distal part (18) of the transverse axis articulation (3) or its bearing elements (40, 41, 42), the opening of which bore (11) receives a closure element (42) which has a locking screw (43) which cooperates with a recess (47) in the bore wall and which has at its two ends key surfaces (48, 50) which are accessible through openings (47, 49).

8. Prosthesis according to Claim 7, **characterized in that** the closure element (42) is formed by the end piece of one of the bearing elements (41, 42).

9. Prosthesis according to one of Claims 2 to 8, **characterized in that** the connecting arrangements (5-8) and the proximal (9) and distal (18) part of the transverse axis articulation (3) can be pushed into one another in the same direction.

## Revendications

1. Prothèse du poignet comprenant un élément carpien (2) à implanter dans le métacarpe, un élément radial (1) à fixer contre l'os de l'avant-bras et une articulation à axe transversal (3) intercalée entre les deux, dont la partie proximale (9) est assemblée à l'élément radial (1), **caractérisée en ce que** deux modes de réalisation de la partie proximale (9) de l'articulation à axe transversal (3) sont disponibles, parmi lesquelles le premier est rigide en soi et le deuxième comporte une articulation supplémentaire (25, 28), et **en ce que** les deux modes de réalisation de la partie proximale (9) de l'articulation à axe transversal (3) sont assemblés ou peuvent être assemblés de manière amovible avec l'élément radial (4) et la partie distale (18) de l'articulation à axe transversal (3).

2. Prothèse selon la revendication 1, **caractérisée en ce que** la partie proximale (9) de l'articulation à axe transversal (3) et l'élément radial (1) sont munis de dispositifs d'assemblage (5-8) transmettant les forces par conjugaison de forme.

3. Prothèse selon la revendication 1 ou 2, **caractérisée en ce que** l'articulation à axe transversal (3) comprend un pivot en T (18, 19), qui forme la partie distale (18) de l'articulation à axe transversal (3).

4. Prothèse selon l'une quelconque des revendications 1 à 3, **caractérisée en ce que** la partie distale (18) de l'articulation à axe transversal (3) comprend les éléments formant paliers (41, 42) de l'articulation à axe transversal (3) et l'assemblage entre la partie distale (18) de l'articulation à axe transversal (3) et la partie proximale (9) de l'articulation à axe transversal (3) est statique.

5. Prothèse selon l'une quelconque des revendications 1 à 4, **caractérisée en ce que** l'angle (α) entre l'axe (13) de l'articulation à axe transversal (3) et une pièce transversale (21) de l'élément carpien (2) n'est pas supérieur à 15°.

6. Prothèse selon la revendication 5, **caractérisée en ce que** l'articulation supplémentaire (25, 28) comporte une cavité articulaire (25), dont la tangente du bord (29) dans le plan palmaire forme avec la direction transversale du radius (37) un angle (β) de 5° à 25°.

7. Prothèse selon l'une quelconque des revendications 1 à 6, **caractérisée en ce que** la partie proximale (9) de l'articulation à axe transversal (3) comporte une forure (11, 12), s'étendant dans le sens ulnaire-radial et destinée à recevoir la partie distale (18) de l'articulation à axe transversal (3) ou ses éléments formant paliers (40, 41, 42), l'orifice de ladite forure recevant un élément de blocage (42), qui comporte une vis de blocage (43), qui coopère avec un évidement (47) dans la paroi de la forure et dont les deux extrémités sont munies de surfaces pour clé à vis (48, 50) accessibles par des orifices (47, 49).

8. Prothèse selon la revendication 7, **caractérisée en ce que** l'élément de blocage (42) est formé par l'extrémité de l'un des éléments formant paliers (41, 42).

9. Prothèse selon l'une quelconque des revendications 2 à 8, **caractérisé en ce que** les dispositifs d'assemblage (5-8) et la partie proximale (9) et la partie distale (18) de l'articulation à axe transversal (3) peuvent coulisser l'un dans l'autre dans la même direction.
